# EUROPEAN PATENT APPLICATION

(11) **EP 4 537 737 A1**
(43) Date of publication of application: **16.04.2025**
(21) Application number: 23203192.2
(22) Date of filing: 12.10.2023
(51) Int. Cl.: A61B 1/06, A61B 1/24, A61B 5/00, A61C 1/08, A61B 90/30, A61B 90/00, G01J 3/50

(54) **EXTRAORAL DENTAL ILLUMINATION SYSTEM**

(71) Applicant: Koninklijke Philips N.V., 5656 AG Eindhoven (NL)
(72) Inventor: JOHNSON, Mark Thomas, Eindhoven (NL); GERHARDT, Lutz Christian, Eindhoven (NL); RMAILE, Amir Hussein, Eindhoven (NL); KOOIJMAN, Gerben, Eindhoven (NL)
(74) Representative: Philips Intellectual Property & Standards

(57) **Abstract**

The subject-matter of the present disclosure relates to an extraoral dental illumination system, EDIS, (102) comprising: an illumination device (302) for illuminating an oral cavity of a the mouth of the subject; and a controller (306) including a processor and storage, the storage having instructions stored thereon that, when executed by the processor, cause the processor to: receive data, the data including a prediction of an oral problem; and determine an imaging setting of the extraoral dental illumination system, from a plurality of imaging settings where each imaging setting is optimised for observing a particular oral problem.

## Description

### FIELD OF THE INVENTION

The subject-matter of the present disclosure relates to an extraoral dental illumination system, EDIS, an oral home care device, and a dental system.

### BACKGROUND OF THE INVENTION

Certain dental or oral problems, e.g. caries, cavities, gingivitis, cancer etc. are easier to detect at different imaging settings. For example, one wavelength of light may be optimal for imaging one oral problem whereas another wavelength of light may be better for imaging another oral problem. Therefore, when a subject is being examined by a dentist, time may be wasted investigating an oral cavity of a subject and some oral problems may be missed or misdiagnosed if observed at an incorrect imaging setting.

Furthermore, it is not safe to always/continuously use certain illumination wavelength (e.g. blue/UV). Continuously capturing data at multiple wavelengths results in long chair times and also huge data sets in the Dental Professionals (DP)-office. For instance, as in a home environment, there is usually sufficient time for imaging at different wavelengths (2x brushing per day for 2 mins over multiple days) to screen for oral conditions and symptoms to obtain insight in a person's oral health status; it would be very beneficial to guide the DP in the office to select suitable wavelengths and/or spectral filter characteristics for the most likely expected condition in order to reduce image acquisition time and image data volume (to enable in office DP workflow optimization and dental chair time reduction). The home or remote imaging system derives the most likely dental condition or symptom which the DP starts to examine and screen for first using, automatically adapted wavelengths and filter settings.

It is an aim of the subject-matter of the present disclosure to improve on the prior art.

### SUMMARY OF THE INVENTION

According to a first aspect of the present invention, there is provided an extraoral dental illumination system, EDIS, comprising: an illumination device for illuminating an oral cavity of a the mouth of the subject; and a controller including a processor and storage, the storage having instructions stored thereon that, when executed by the processor, cause the processor to: receive data, the data including a prediction of a oral problem; and determine an imaging setting of the extraoral dental illumination system, from a plurality of imaging settings where each imaging setting is optimised for observing a particular oral problem.

In an embodiment, the EDIS comprises an imaging system, wherein the controller is configured to capture an image using the imaging system and the illumination device based on the imaging settings. More specifically, this may be based on measurements with an intraoral dental (home or remote device) or other input such as questionnaires. The EDIS can be a standalone device or part of a dental assessment system or dental examination system.

The EDIS of Claim 2, wherein the data comprises a plurality of wavelengths for both the illumination device and/or a filter setting of the imaging system, each oral problem associated with one or more wavelengths of the plurality of wavelengths. The term one or more is used because there may be a spectral band of wavelengths, rather than a single wavelength, and the oral problem may also be represented best using a mix of more than one wavelength, e.g. white light.

In an embodiment, each wavelength of the plurality of wavelengths is selected from a list of wavelengths including: 500 to 600nm when the oral problem is an enamel lesion; caries 520nm of 1620nm when oral problem is caries; 450-500nm when the oral problem involves pre-cancerous soft tissue abnormalities detectable by nicotinamide adenine dinucleotide ; 500-550nmoral problem when the oral problem involves pre-cancerous soft tissue abnormalities detectable by flavin adenine dinucleotide; 600-700 when the oral problem is a dentine lesion; 630nm when the oral problem is plaque and tartar; 410-440nm or 550-600nm when the oral problem is gingivitis; and 1950nm when enamel demineralization/fluorosis. The plurality of wavelengths may be between 300 and 2200 nm.

In an embodiment, the illumination source is configured to flash pulses of light in a sequence and the camera is synchronised temporally with the flashes, wherein one or more of the pulses of light being at a wavelength from the imaging setting.

In an embodiment, the data includes a position and/or orientation of the oral problem in a subject's mouth, the controller is further configured to: generate a map of the mouth including the oral problem at the position and/or orientation superimposed onto a dental structure; detecting a region of interest for a dentist using the EDIS; registering the region of interest with the map; and controlling the wavelength to be the wavelength associated with the oral problem when the oral problem in the map appears in the region of interest and to be another of the plurality of wavelengths when the oral problem does not appear in the region of interest. The term dental structure may refer to one or more of a dental arch, a jaw, a section of teeth, etc.

In an embodiment, the detecting the region of interest (ROI) for a dentist using the EDIS comprises at least one of: tracking, using a gaze tracking module, a gaze of the dentist; and recognising specific foreign dental device features appearing in the image.

In an embodiment, the detecting the region of interest for the dentist using the EDIS comprises: monitoring images captured from the imaging device; and identifying a light pattern or spot light from the illumination device in the images using a feature detection model. The feature detection module may be either a segmentation module or an image feature recognition module. The spot light may be a directed light beam.

In an embodiment, the data comprises sensor data from an intraoral home device, subject data and/or subject questionnaire results.

According to an aspect of the present invention, there is provided an oral home care device, comprising: a sensor; and a controller including a processor and storage, the storage having instructions stored thereon that, when executed by the processor, cause the processor to: monitoring parameters sensed by the sensor; oral problem; generating data; and sending a signal to an extraoral dental illumination system, EDIS, the signal including the data to enable the EDIS to determine an imaging setting, from a plurality of imaging settings where each imaging setting is optimised for observing a particular oral problem.

In an embodiment, the instructions cause the processor to predict a oral problem based on the sensed parameters, wherein the generated data includes the oral problem.

In an embodiment, the data comprises a plurality of wavelengths for both an illumination device and a filter of a camera uses for capturing an image of the oral problem, each oral problem associated with one or more wavelengths of the plurality of wavelengths. The term one or more is used because there may be a spectral band of wavelengths, rather than a single wavelength, and the oral problem may also be represented best using a mix of more than one wavelength, e.g. white light.

In an embodiment, each wavelength of the plurality of wavelengths is selected from a list of wavelengths including: 500 to 600nm when the oral problem is an enamel lesion; caries 520nm of 1620nm when oral problem is caries; 450-500nm when the oral problem involves pre-cancerous soft tissue abnormalities detectable by nicotinamide adenine dinucleotide ; 500-550nmoral problem when the oral problem involves pre-cancerous soft tissue abnormalities detectable by flavin adenine dinucleotide; 600-700 when the oral problem is a dentine lesion; 630nm when the oral problem is plaque and tartar; 410-440nm or 550-600nm when the oral problem is gingivitis; and 1950nm when enamel demineralization/fluorosis. The plurality of wavelengths may be between 300 and 2200 nm.

In an embodiment, the data further comprises subject data and/or subject questionnaire results.

In an embodiment, the sensor comprises a sensor from a list of sensors including: an inertial measurement unit for monitoring a vibrations of the oral home care device; a force sensor for monitoring surface friction force; wherein the oral home care device is a toothbrush, a force sensor in the toothbrush to monitor force or pressure applied while brushing; and electrodes for monitoring galvanic skin response.

In an embodiment, the predicting a oral problem based on the sensed parameters comprises: computing an average for the monitoring parameter over a brushing time; comparing the average to a one or more thresholds; and identifying the oral problem based on the comparison.

In an embodiment, the sensor comprises, or may be, a sensor from a list of sensors: including a multi- or hyper- spectral image sensor array or single pixel sensor for monitoring spectral bands; an image sensor for monitoring colour channels in red, green, and blue, images; and a fluorescence sensor for imaging fluorescent signals of biomolecules.

In an embodiment, the predicting the oral problem based on the sensed parameter of the sensor comprises: generating (light) intensity of the parameter as a function of the imaging setting; determining locations of changes in intensity; determining orientations of changes in intensity; and identifying the oral problem and the location and orientation associated with the oral problem based on the determined locations and orientations of changes in intensity.

In an embodiment, where more than one oral problem is predicted, the method further comprises: determining a rate of change of the sensed parameter over time; and ranking the oral problems based on a magnitude of the rate of change. The rate of change may be a time rate of change, e.g. a time differential.

According to an aspect of the present invention, there is provided a dental assessment system comprising: the extraoral dental illumination system of any preceding aspect or embodiment; and the oral home care device of any preceding aspect or embodiment.

According to an aspect of the present invention, there is provided a method of controlling an EDIS, comprising: receiving data, the data including a prediction of an oral problem; determining an imaging setting, from a plurality of imaging settings where each imaging setting is optimised for observing a particular oral problem; and capturing an imaging using an imaging system and an illumination device of the dentist device based on the imaging setting.

According to an aspect of the present invention, there is provided a method of controlling an oral home care device, comprising: monitoring parameters sensed by a sensor of the oral home care device; predicting an oral problem based on the sensed parameters; generating data including the prediction of the oral problem; and sending a signal to a dentist device, the signal including the data to enable the dentist device to determine an imaging setting, from a plurality of imaging settings where each imaging setting is optimised for observing a particular oral problem.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying figures, in which:
Fig. 1 shows a block diagram of a dental assessment or examination system according to one or more embodiments;
Fig. 2 shows a schematic diagram of an intraoral dental (home, remote) device according to one or more embodiments;
Figs. 3A to 3C show perspective views of illustrations of example extraoral dental illumination systems, EDIS, three different embodiments;
Fig. 4 shows a schematic diagram of the extraoral dental (home, remote) illumination system, EDIS, of Fig. 3; and
Fig. 5 shows a block diagram of a method used by the extraoral dental (home, remote) illumination system, EDIS, to capture images of an oral problem.

### DESCRIPTION OF EMBODIMENTS

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

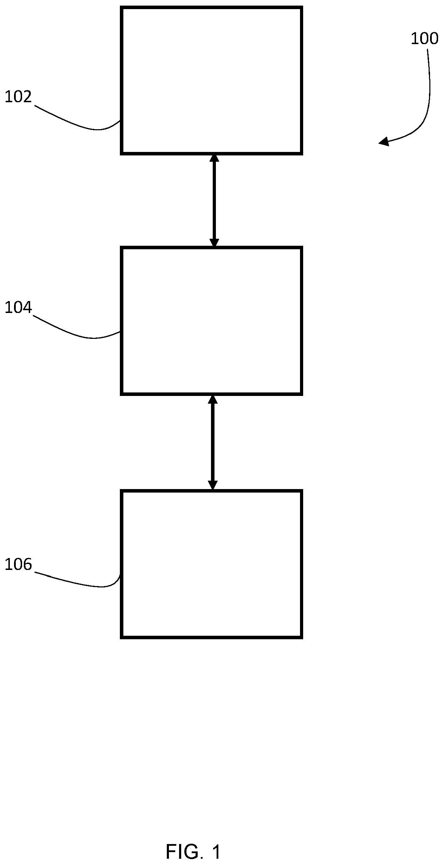

With reference to Fig. 1, a dental assessment system 100 includes an oral home care device device 102, a control system 104, and an extraoral dental professional illumination system, EDIS 106.

The control system 104 includes at least a communication unit to communicate between the oral home care device 102 and the EDIS 106.

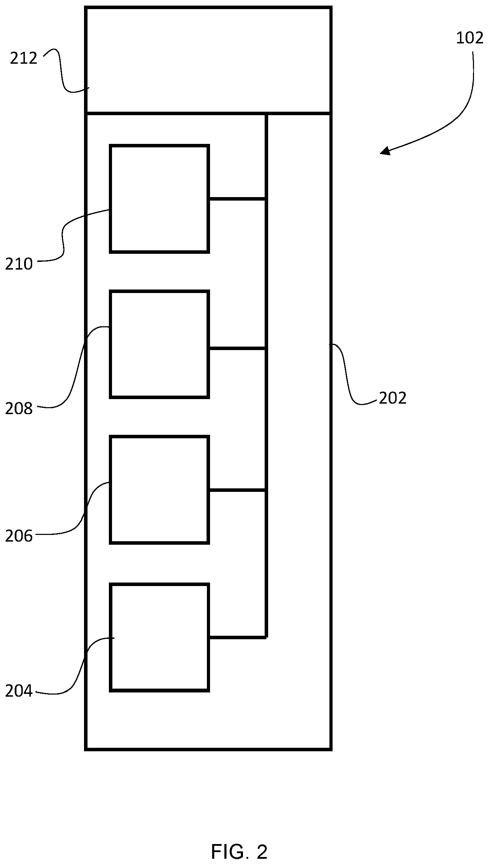

With reference to Fig. 2, the oral home care device 102 includes a handle 202, a power storage unit 204, a communication unit 206, a controller 208, a sensing system 210, and optionally an operational head 212. The operational head 212 may be a toothbrush head when the oral home care device is a toothbrush, or powered tooth brush (PTB), for example, or a nozzle when the device is an irrigator or flossing device. The oral home care device 102 may be used by the subject at home or in any remote/retail environment.

The power storage unit 204 may be a battery, for example a secondary battery. The power storage unit 204 may provide power for the communication unit 206, the controller 208, and the sensing system 210.

The communication unit 206 may be communicatively link to the control system 104 (Fig. 1), or alternatively, directly with the EDIS 106 (Fig. 1). The communication unit 206 may be configured to send signals generated by the controller.

The controller and the communication unit may be provided within the handle or remote from the handle. The controller includes storage and a processor. The storage includes electronic data storing instructions that when executed by the processor cause the processor to perform a computer-implemented method. In this way, the storage acts as non-transitory computer-readable media. The instructions may also be saved to the storage when they are provided as transitory computer-readable media.

The sensing system 210 includes one or more sensors, for example, to generate data for determining a prediction of an oral or dental condition and providing a control signal for selection of imaging settings to the EDIS.

The instructions on the controller storage cause the processor to monitor parameters sensed by the one or more sensors, predict a oral problem based on the sensed parameters, generate data including the prediction of the oral problem, generate a signal for the communication unit 206 to send to the EDIS, via the control system 104. The signal includes the data to enable the EDIS to determine an imaging setting, from a plurality of imaging settings, where each imaging setting is optimised for observing a particular oral problem.

The oral problems that are to be detected include enamel lesions, caries, soft cancerous oral tissue , dental lesions, plaque, tartar, gingivitis, and enamel demineralization/fluorosis.

The imaging settings preferably includes one or more wavelengths where the oral problem is most visible, or optimal. The term one or more is used because there may be a spectral band of wavelengths, rather than a single wavelength, and the oral problem may also be represented best using a mix of more than one wavelength, e.g. white light. In addition, certain oral problems may show up better at certain colour temperatures. However, other imaging settings may also be provided. Such other imaging settings include a filter setting of a camera used for capturing an image of the oral problem, and also characteristics such as auto-zoom, contrast, frame rate, resolution, light intensity, etc.

Each wavelength of the plurality of wavelengths is selected from a list of wavelengths including: 500 to 600nm when the oral problem is an enamel lesion; caries 520nm of 1620nm when oral problem is caries; 450-500nm when the oral problem involves detecting nicotinamide adenine dinucleotide; 500-550nm when the oral problem involves detecting flavin adenine dinucleotide; 600-700 when the oral problem is a dentine lesion; 630nm when the oral problem is plaque and tartar; 410-440nm or 550-600nm when the oral problem is gingivitis; and 1950nm when enamel demineralization/fluorosis.

The wavelength information may be identified in various ways. For example, the wavelengths may be obtained from a look-up table when the controller has predicted a oral problem. Alternatively, where the sensing system includes a camera and illumination means, one or more wavelengths used by the illumination means when observing the oral problem may be identified by the illumination system.

The data may also include subject data and/or results from a subject questionnaire. These aspects may be included at the control system 104. The subject data may have been entered by a dentist after one or more previous visits by the subject to a dental surgery. The questionnaire may be provided to subject at the surgery and input using a user interface and sent to the control system 104, for example. The questionnaire may help to identify, for examples, location information, levels of pain or discomfort, etc.

The one or more sensors can be at least one sensor from a list of sensors including: an inertial measurement unit for monitoring a vibration of the oral home care device; a force sensor for monitoring surface friction force; wherein the oral home care device is a toothbrush, a force sensor in the toothbrush to monitor force or pressure applied while brushing; and electrodes for monitoring galvanic skin response. When the sensed parameter is obtained using one or more of these sensors, the controller may compute an average of the monitored parameter over a brushing time, compare the average to one or more thresholds, and identify the oral problem based on the comparison. For example, a change in average platen reaction force may indicate tooth mobility or painful areas. Similarly, bristle friction changes may indicate plaque versus non-plaque areas. In this way, the one or more thresholds may include a different threshold or a group of thresholds for each potential oral problem.

Other types of sensors may also be provided, such as various imaging sensors. For example, the one or more sensors may include at least one sensor from a list of sensors including a multi-or hyper- spectral image sensor array or single pixel sensor for monitoring spectral light bands, preferably in near infrared (NIR) wavelength range; an image sensor for monitoring colour channels in red, green, and blue, images; and a fluorescence sensor for imaging fluorescent signals of biomolecules. The sensor may sense the parameter which may be characterised by light intensity, polarization or light colour of each image sensor pixel.

In this way, the controller may be configured to generate intensity of the parameter as a function of the imaging setting; determining locations of changes in intensity; determining orientations of changes in intensity; and identifying the oral problem and the location and orientation associated with the oral problem based on the determined locations and orientations of changes in intensity. The intensity may be determined as pixel intensity as a function of wavelength, λ: I(λ). The location of the intensity may be determined by pixel intensity as a function of position of the device in the mouth in cartesian coordinates, (x, y, z) and orientation relative to the oral problem (α, β, γ for roll, yaw, and pitch, angle of the oral device). In order to determine these positional coordinates, the sensor may be an internal measurement unit including an accelerometer and a gyro, for example. Furthermore, the controller may include a feature detection module for detecting features in the images using various known means, e.g. a trained convolutional neural network.

It should be noted that there may be more than one oral problem for a dentist to investigate. The controller may be configured to determine a rate of change of the sensed parameter over time and rank the oral problems based on a magnitude of the rate of change. This may be achieved by determining, for each oral problem, a magnitude of change of the sensed parameters, either image or non-image. The largest change can be taken to mean the most severe problem and the smallest change can be taken to mean the least severe problem. If the data includes subject data or questionnaire data, for example, a probability for certain problems can be added.

An example ranking order could be: 1) caries (on occlusal side of upper right premolar), 2) gingivitis (lower jaw), and 3) tartar/calculus (on upper left canine).

Using the oral home care device may be summarised as a method of controlling an oral home care device, the method comprising: monitoring parameters sensed by a sensor of the oral home care device; predicting a oral problem based on the sensed parameters; generating data including the prediction of the oral problem; and sending a signal to a dentist device, the signal including the data to enable the dentist device to determine an imaging setting, from a plurality of imaging settings where each imaging setting is optimised for observing a particular oral problem.

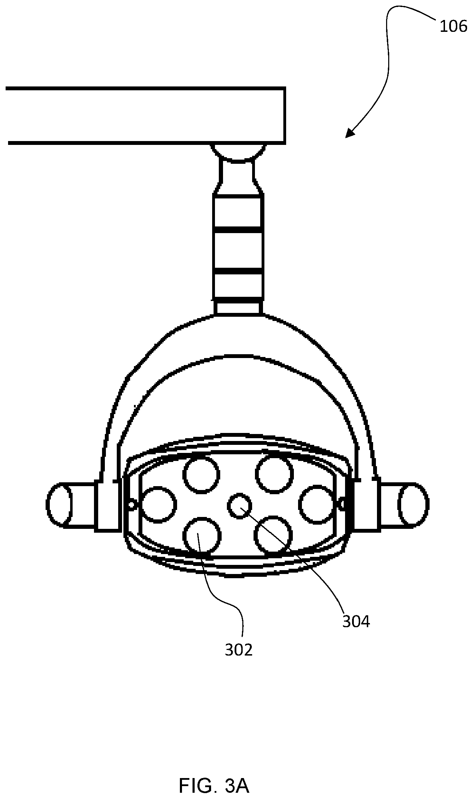

With reference to Figs. 3A to 3C, the extraoral dental illumination system, EDIS, includes 106 an illumination source 302 for illuminating an oral cavity of a mouth of a patient, or subject, and optionally an imaging system 304.

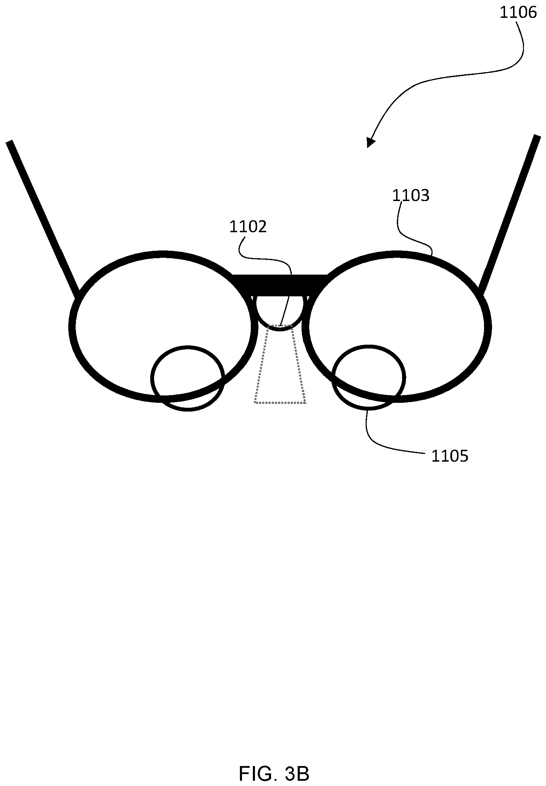

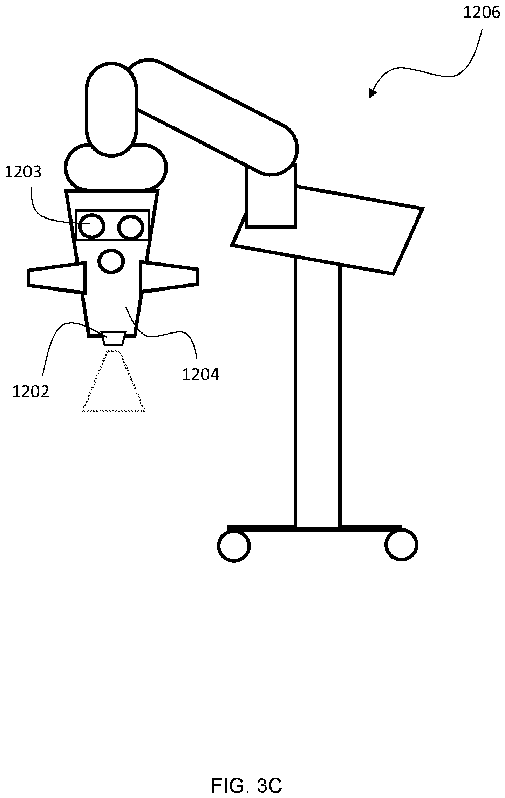

The EDIS 106 may be one or more of: a dental office lighting systems with overhead light with integrated camera (Fig. 3A), medical loupe with in-built multi-wavelength LED light that includes a selectable daylight or violet light for hands-free general or fluorescent illumination and loupe-mounted camera (Fig. 3B), dental surgery microscope with multifunctional manual or automatic mode control (Fig. 3C) which includes adjusting the light intensity, activating all the augmented visualization modes and turning on the spotlight and capture modes, and dentist using VR/AR glasses.

With reference to Fig. 3A, the EDIS includes the illumination source 302 and the and the imaging system 304.

With reference to Fig. 3B, the EDIS, in the form of medical loupes 1106, includes the illumination source 1102, spectacles 1103 and magnifying loupes 1105.

With reference to Fig. 3C, the EDIS in the form of the dental microscope 1206, includes the illumination source 1202, the imaging system 1204 in the form of a microscope, and binocular eye pieces 1203.

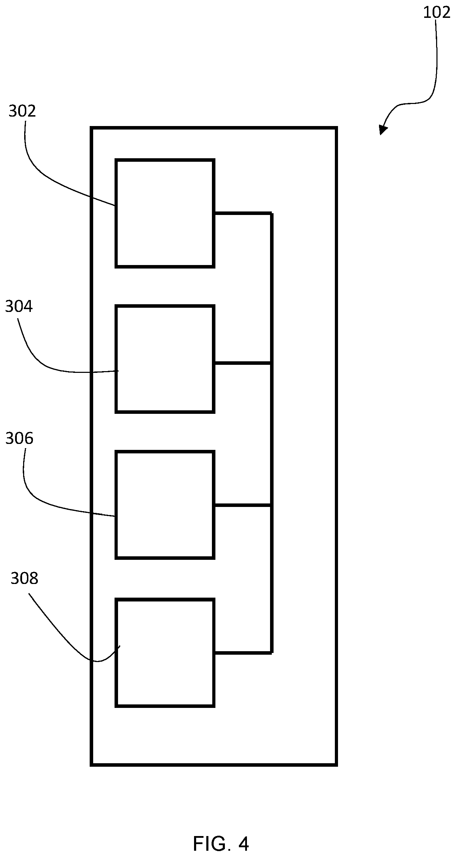

With reference to Figs. 3A to 4, the EDIS 106 also includes a controller 306. The controller includes a processor and storage, the storage having instructions stored thereon that, when executed by the processor cause the processor to receive data, the data including a prediction of a oral problem; and determine an imaging setting of the extraoral dental illumination system, from a plurality of imaging settings where each imaging setting is optimised for observing a particular oral problem. Again, the storage may be non-transitory computer-readable media. The instructions may be provided as a download when provided as transitory computer-readable media.

The EDIS 106 may include a power unit 308 for powering the controller, the imaging system, and the illumination source. The power unit may be a battery. The battery may be a secondary battery, for example.

The imaging system 304 may include a camera. The controller may be configured to capture an image using the imaging system and the illumination device based on the imaging settings.

The controller may configure the illumination source to emit light at one or more wavelengths associated with the oral problem that the subject has. This can be continued for the entire dental procedure. Alternatively, different wavelengths of light can be used at different parts of the dental procedure. For instance, when the dentist is focusing on the oral problem, the associated one or more wavelengths can be used, and otherwise different wavelengths may be used for the remainder of the procedure, e.g. white light.

The controller may configure the illumination source to flash pulses of light in a sequence. The imaging system may be synchronised temporally with the flashes. For instance, one of light flashes may be at a wavelength, or wavelengths, associated with one oral problem for example 500 to 600nm when the oral problem is an enamel lesion, another flash may be at a wavelength, or wavelengths, associated with another oral problem for example 410-440nm when the oral problem is gingivitis, and another wavelength, or wavelengths, for example white light, may be other for a third flash.

While the imaging setting may be obtained from a look-up table. In other embodiments, the imaging setting may be the same imaging settings used to capture images of the oral problem using the oral home care device.

When the data includes a position and/or orientation of the oral problem in a subject's mouth, the controller is configured to generate a map of the mouth including the oral problem at the position and/or orientation superimposed onto a dental structure; detecting a region of interest for a dentist using the EDIS; registering the region of interest with the map; and controlling the wavelength to be the wavelength associated with the oral problem when the oral problem in the map appears in the region of interest and to be another of the plurality of wavelengths when the oral problem does not appear in the region of interest. In this way, the EDIS dynamically adapts the imaging settings of the imaging system and/or illumination system depending on its position within the mouth and its proximity to the oral problem. The term "dental structure" may be used to mean, for example, a jaw, a dental arch, etc. Coordinate positions may be included for each part of the dental structure, and also the oral problem on the dental structure.

The controller and EDIS may include a gaze tracking module. The gaze tracking module may use images from the imaging system 304. How the gaze tracking works will depend on what type of EDIS is being used. For example, if loupes are used, the imaging system can monitor where the dentist is looking based on features that exist in the images. If other types of EDIS are used, a position of a spotlight from the illumination system within an image, or even the presence of dental device features within the images, can be used. For example, it a dental examination mirror or poking tool is detected within the image, its location provides a good indication as to where the dentist is looking. Image detection may be carried out using a variety of algorithms based on a trained convolutional neural network, for example.

In this way, the detecting the region of interest for the dentist using the EDIS comprises monitoring images captured from the imaging device; and identifying a light pattern or spot light (incl. focused or directed beam) from the illumination system in the images using a feature detection model.

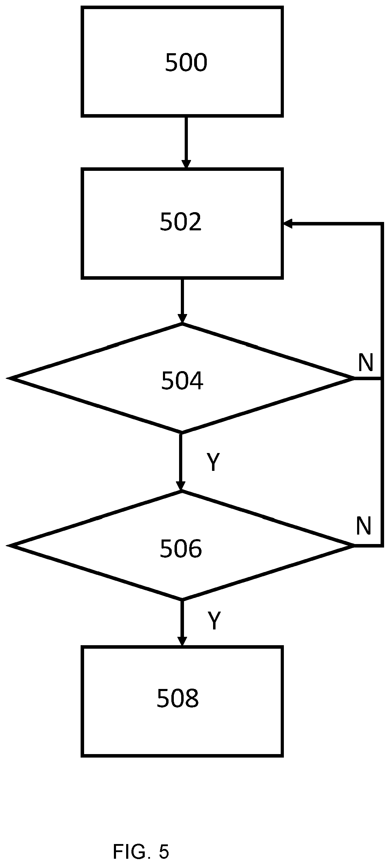

With reference to Fig. 5, the method used by the EDIS to capture images of the oral problem may be envisaged as follows.

At step 500, the map of the subject's mouth is generated showing the most likely oral problem and required corresponding imaging setting from the oral home care device (typically a home device or oral care device like a toothbrush).

At step 502, the dentist starts using the EDIS, optionally at a random default setting, and the dentist or software method (automatically) checks whether the regional of interest (gaze), directed light beam, or dental device feature is in the field of view of the imaging system. This can be identified from the live image capture by the imaging system.

At step 504, the controller checks to see whether the gaze, spotlight, or dental device feature is detected in a field of view of the image. If no, the method reverts to step 502. If yes, the method proceeds to step 506.

At step 506, the location of the oral problem in the field of view is compared to the corresponding location from the home using the intraoral map with predetermined oral conditions generated by the oral home care device. If no, the method repeats step 502. It yes, the method proceeds to step 508.

At step 508, the imaging setting of the EDIS illumination system is set according to the dental condition. For example, the wavelength used on the oral home care device may be set as the setting of the illumination system. Where the imaging system is provided on the EDIS, an image is captured using the imaging setting where applicable, and the images are saved by the controller. The images may be sent to the control system.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. An extraoral dental illumination system, EDIS, (102) comprising:
an illumination device (302) for illuminating an oral cavity of a mouth of a subject; and
a controller (306) including a processor and storage, the storage having instructions stored thereon that, when executed by the processor, cause the processor to:
receive data, the data including a prediction of a oral problem; and
determine an imaging setting of the extraoral dental illumination system, from a plurality of imaging settings where each imaging setting is optimised for observing a particular oral problem.

2. The EDIS of claim 1, comprising an imaging system (304), wherein the controller is configured to capture an image using the imaging system and the illumination device based on the imaging settings.

3. The EDIS of Claim 2, wherein the data comprises a plurality of wavelengths for both the illumination device and/or a filter setting of the imaging system, each oral problem associated with one or more wavelengths of the plurality of wavelengths.

4. The EDIS of Claim 3, wherein each wavelength of the plurality of wavelengths is selected from a list of wavelengths including: 500 to 600nm when the oral problem is an enamel lesion; caries 520nm of 1620nm when oral problem is caries; 450-500nm when the oral problem involves nicotinamide adenine dinucleotide ; 500-550nm when the oral problem involves flavin adenine dinucleotide; 600-700 when the oral problem is a dentine lesion; 630nm when the oral problem is plaque and tartar; 410-440nm or 550-600nm when the oral problem is gingivitis; and 1950nm when enamel demineralization/fluorosis.

5. The EDIS of any of Claims 2 to 4, wherein the data includes a position and/or orientation of the oral problem in a subject's mouth, the controller is further configured to:
generate a map of the mouth including the oral problem at the position and/or orientation superimposed onto a dental structure;
detecting a region of interest for a dentist using the EDIS;
registering the region of interest with the map; and
controlling the wavelength to be the wavelength associated with the oral problem when the oral problem in the map appears in the region of interest and to be another of the plurality of wavelengths when the oral problem does not appear in the region of interest.

6. The EDIS of Claim 5, wherein the detecting the region of interest for a dentist using the EDIS comprises at least one of:
tracking, using a gaze tracking module, a gaze of the dentist; or
recognising specific foreign dental device features appearing in the image, or both

7. The EDIS of Claim 6, wherein the detecting the region of interest for the dentist using the EDIS comprises:
monitoring images captured from the imaging device; and
identifying a light pattern or spotlight from the illumination device in the images using a feature detection model.

8. An oral home care device (106), comprising:
a sensor; and
a controller (208) including a processor and storage, the storage having instructions stored thereon that, when executed by the processor, cause the processor to:
monitoring parameters sensed by the sensor;
generating data based on the monitored parameters; and
sending a signal to an extraoral dental illumination system, EDIS, (102) the signal including the data to enable the EDIS to determine an imaging setting, from a plurality of imaging settings where each imaging setting is optimised for observing a particular oral problem.

9. The oral home care device of Claim 8, wherein the instructions cause the processor to: predict a oral problem based on the sensed parameters, wherein the generated data includes the oral problem.

10. The oral home care device of Claim 9, wherein the data comprises a plurality of wavelengths for both an illumination device and a filter of a camera used for capturing an image of the oral problem, each wavelength of the plurality of wavelengths being optimal for capturing an image of a oral problem.

11. The oral home care device of Claim 9 or Claim 10, wherein the sensor comprises a sensor from a list of sensors including: an inertial measurement unit for monitoring a vibrations of the oral home care device; a force sensor for monitoring surface friction force; wherein the oral home care device is a toothbrush, a force sensor in the toothbrush to monitor force or pressure applied while brushing; and electrodes for monitoring galvanic skin response.

12. The oral home care device of Claim 11, wherein the predicting a oral problem based on the sensed parameters comprises:
computing an average for the monitoring parameter over a brushing time;
comparing the average to a one or more thresholds; and
identifying the oral problem based on the comparison.

13. The oral home care device of any of Claims 9 to 12, wherein the sensor comprises a sensor from a list of sensors: including a multi- or hyper- spectral image sensor array or single pixel sensor for monitoring spectral bands; an image sensor for monitoring colour channels in red, green, and blue, images; and a fluorescence sensor for imaging fluorescent signals of biomolecules.

14. The oral home care device of Claim 13, wherein the predicting the oral problem based on the sensed parameter of the sensor comprises:
generating intensity of the parameter as a function of the imaging setting;
determining locations of changes in intensity;
determining orientations of changes in intensity; and
identifying the oral problem and the location and orientation associated with the oral problem based on the determined locations and orientations of changes in intensity.

15. The intraoral dental device of any of Claims 9 to 14, where more than one dental problem is predicted, the method further comprises:
determining a rate of change of the sensed parameter over time; and
ranking the dental problems based on a magnitude of the rate of change.
